# EUROPÄISCHE PATENTSCHRIFT

(11) **EP 3 705 457 B1**
(45) Veröffentlichungstag und Bekanntmachung des Hinweises auf die Patenterteilung: **18.02.2026**
(21) Anmeldenummer: 20160922.9
(22) Anmeldetag: 04.03.2020
(51) Int. Cl.: C02F 1/00, B01D 17/02, G01N 33/18, C02F 1/40, C02F 3/12

(54) **REINIGUNGSANLAGE, INSBESONDERE FÜR ABWASSER, ZUR ABSCHEIDUNG VON LEICHTFLÜSSIGKEIT UND/ODER FETTEN UND VERFAHREN ZUM BETRIEB DIESER REINIGUNGSANLAGE**
CLEANING INSTALLATION, IN PARTICULAR FOR WASTE WATER, FOR SEPARATING LIGHT LIQUID AND / OR FATS AND METHOD FOR OPERATING THIS CLEANING INSTALLATION
INSTALLATION DE NETTOYAGE, EN PARTICULIER POUR EAUX USÉES, PERMETTANT DE SÉPARER UN LIQUIDE LÉGER ET/OU DES GRAISSES ET PROCÉDÉ DE FONCTIONNEMENT DE CETTE INSTALLATION DE NETTOYAGE

(30) Priorität: 05.03.2019 DE 102019105482
(43) Veröffentlichungstag der Anmeldung: 09.09.2020
(73) Patentinhaber: ACO Ahlmann SE & Co. KG, 24782 Büdelsdorf (DE)
(72) Erfinder: SCHÄFER, Manfred, 67685 Weilerbach (DE); SCHMITZ, Marc, 68239 Mannheim (DE)
(74) Vertreter: Meissner Bolte Partnerschaft mbB

(56) Entgegenhaltungen:
- WO-A1-2009/011660
- US-A- 2 670 848
- US-A- 5 122 280

## Beschreibung

Die Erfindung betrifft eine Reinigungsanlage, insbesondere für Abwasser, zur Abscheidung von Leichtflüssigkeit und/oder Fetten. Die Reinigungsanlage umfasst einen oder mehrere, zumindest ein Abscheidevolumen und ein Speichervolumen bildenden bzw. bildende Abscheidebehälter. Das Abscheidevolumen weist mindestens einen Zulauf für zu reinigende Flüssigkeiten, insbesondere für Abwasser auf. Das Abscheidevolumen und das Speichervolumen sind über einen Abscheider zum Abscheiden von Leichtflüssigkeiten und/oder Fetten fluidisch verbunden. Ferner ist eine Hebeanlage zum Fördern von Flüssigkeit aus dem Speichervolumen vorgesehen.

Die Erfindung betrifft ferner ein Verfahren zum Betrieb einer Reinigungsanlage, insbesondere für Abwasser, zur Abscheidung von Leichtflüssigkeit und/oder Fetten. Die Erfindung betrifft insbesondere ein Verfahren zum Betrieb der vorstehend genannten Reinigungsanlage. Die Reinigungsanlage umfasst einen oder mehrere, zumindest ein Abscheidevolumen und ein Speichervolumen bildenden bzw. bildende Abscheidebehälter, die über einen Abscheider zum Abscheiden von Leichtflüssigkeiten und/oder Fetten fluidisch verbunden sind. Zu reinigende Flüssigkeiten, insbesondere Abwasser, werden dem Abscheidevolumen zugeführt. Verunreinigungen, insbesondere in Form von Leichtflüssigkeiten und/oder Fetten, werden über den Abscheider abgeschieden und die gereinigten Flüssigkeiten werden im Speichervolumen gesammelt.

Reinigungsanlagen der eingangs genannten Art sind aus dem Stand der Technik hinlänglich bekannt. So beschreibt beispielsweise DE 199 54 437 A1 eine Abwasserreinigungsanlage zur Abscheidung von Leichtflüssigkeit mit einem vorgeordneten Schlammfangteil, in das Abwasser einleitbar ist, und einem nachgeordneten Speicherbecken zum Sammeln von gereinigtem Wasser. Das Schlammfangteil ist über ein Überlaufrohr mit dem Speicherbecken fluidisch verbunden. Ein Leichtflüssigkeitsabscheider, dessen Ablauf mit dem Überlaufrohr verbunden ist, ist dazu vorgesehen, Leichtflüssigkeitsanteile von dem zugeführten Abwasser abzutrennen. Das gereinigte Abwasser kann mit Hilfe einer Pump- oder Hebeleitung aus dem Speicherbecken abgepumpt werden. Der Zulauf zum Schlammfangteil kann einen selbsttätig verschießenden Zulaufverschluss aufweisen.

Ferner offenbart US 5 122 280 A eine Abscheidevorrichtung zum Trennen von Öl aus Wasser mit einem Vorratsbehälter und einem Abscheidetank, der mit dem Vorratsbehälter fluidisch verbunden ist. Die Abscheidevorrichtung weist eine Pumpe und eine Sensorsonde auf, die mit einer Steuerung signalgekoppelt sind.

Des Weiteren beschreibt WO 2009/011660 A1 eine Vorrichtung zur Abscheidung von Fetten, die einen Auffangbehälter und einen Abscheidebehälter aufweist. Der Abscheidebehälter ist mit Sensoren ausgestattet, die das Vorhandensein von Wasser oder Fett erkennen. Abhängig davon, ob die Sensoren Wasser oder Fett erkennen, wird entweder ein Ventil geöffnet und Wasser abgelassen oder eine Pumpe aktiviert und Fett in einen Vorratsbehälter abgepumpt.

Insbesondere für Leichtflüssigkeitsabscheider werden selbsttätige Verschlusseinrichtungen regulatorisch gefordert. Bisher werden hierfür typischerweise Zulaufsperren oder Schwimmer am Einlaufelement verwendet, die den Zufluss zum Leichtflüssigkeitsabscheider verschließen, wenn der maximale Betriebswasserstand erreicht ist. Meistens werden sogenannte Schwimmer verwendet, die den Ablauf bei Erreichen der maximal erlaubten Speichermenge an Leichtflüssigkeit durch Aufsetzen auf den Schwimmerabschluss verschließen. Diese Schwimmer sind auf die Dichte der Leichtflüssigkeit tariert und reagieren auf die Dichte der sie umgebenden Flüssigkeit (Wasser oder Leichtflüssigkeit) und damit auf die Lage der Trennschicht zwischen abgeschiedener Leichtflüssigkeit und Wasser.

In US 2 670 848 A ist beispielsweise eine Trennvorrichtung zum Trennen von Öl aus Wasser beschrieben, die einen Trenntank mit einem Zulauf und einem Ablauf aufweist. In dem Trenntank ist ein Abschöpftank mit einem integrierten Schwimmer angeordnet, der den Abschöpftank in zwei Bereiche unterteilt.

Die bekannten Zulaufsperren reagieren konstruktionsbedingt nur auf den vorliegenden Betriebswasserspiegel und nicht auf die vorhandene Speichermenge an Leichtflüssigkeit. Somit wird die Verschlusseinrichtung nur dann ausgelöst, wenn der Betriebswasserspiegel über einen vorgegebenen Pegel steigt. Dies kann beispielsweise dadurch verursacht werden, dass ein starkes Niederschlagsereignis, welches den Bemessungswert übersteigt, eintritt oder der Abscheider aufgrund von Rückstau oder einem verblockten Koaleszenzelement nicht den vorgesehenen freien Ablauf bereitstellt.

Die bekannten Schwimmer sind im Allgemeinen im Bereich eines Ablaufs konstruktiv vorgesehen und verschließen den Abscheider, wenn die maximale Speichermenge an Leichtflüssigkeit erreicht ist. Hierzu sind sie auf die Dichte der maßgeblich vorkommenden Leichtflüssigkeit tariert bzw. liegen hinsichtlich ihrer Dichte geringfügig darüber. Schwimmer reagieren immer nur auf die Dichte der sie unmittelbar umgebenden Flüssigkeit. Wenn verschiedene Flüssigkeiten unterschiedlicher Dichten vorhanden sind, kann der Fall eintreten, dass Trennschichten entstehen, die sich nicht in der unmittelbaren Umgebung des Schwimmers befinden. Sollte sich der Schwimmer im Bereich der Trennschicht befinden, so reagiert er auf das Mittel der ihn umgebenden Flüssigkeiten entsprechend der Trennschichthöhe. Zudem ist der Schwimmer immer nur auf eine Leichtflüssigkeit tariert, so dass der Schwimmer bei Anwesenheit mehrerer Flüssigkeiten mit unterschiedlichen Dichten im Allgemeinen nur unzureichend reagiert. Zudem gibt es das inhärente Problem, dass die Dichte des Schwimmers durch Verunreinigungen verändert werden kann. Dies kann dazu führen, dass der Schwimmer früher den Abscheider verschließt bzw. bereits so schwer wird, dass seine Dichte im Bereich der Dichte des Wassers liegt. In diesem Fall kann es dazu kommen, dass der Schwimmer den Ablauf bereits im Regelbetrieb verschließt.

Schwimmer reagieren weiterhin empfindlich auf die Stärke des Zuflusses. Sollte eine Reinigungsanlage aufgrund eines Starkregenereignisses, einer fehlerhaften Auslegung oder eines zusätzlichen Zuflusses beispielsweise über eine weitere Fläche überlastet werden, so kann der Fall eintreten, dass Wasser über das Koaleszenzelement zum Ablauf fließt. Dabei erfährt der Schwimmer eine zusätzliche Kraft von oben. Diese Kraftkomponente und die Tatsache, dass sich ein großer Sog aufgrund des hohen Durchflusses einstellen kann, kann dazu führen, dass der Schwimmer nach unten gezogen wird und den Abscheider verschließt, obwohl die Anlage weiterhin im Regelbetrieb betrieben werden könnte.

Es ist Aufgabe der Erfindung, eine Reinigungsanlage der eingangs genannten Art bzw. ein Verfahren zum Betrieb einer derartigen Reinigungsanlage anzugeben, bei der die vorstehend beschriebenen Nachteile zumindest zum Teil vermieden werden können.

Diese Aufgabe wird gelöst durch eine Reinigungsanlage mit den Merkmalen des Anspruchs 1 bzw. durch ein Verfahren zum Betrieb einer derartigen Reinigungsanlage mit den Merkmalen des Anspruchs 9.

Vorteilhafte Ausgestaltungen der Erfindung sind Gegenstand der Unteransprüche.

Eine Reinigungsanlage, insbesondere für Abwasser, zur Abscheidung von Leichtflüssigkeit und/oder Fetten, umfasst ein oder mehrere Abscheidebehälter, der bzw. die zumindest ein Abscheidevolumen und ein Speichervolumen bilden. Das Abscheidevolumen weist einen Zulauf für zu reinigende Flüssigkeiten, insbesondere für Abwasser auf. Das Abscheidevolumen und das Speichervolumen sind über einen Abscheider zum Abscheiden von Leichtflüssigkeiten und/oder Fetten fluidisch verbunden. Ferner ist eine Hebeanlage zum Fördern von Flüssigkeit aus dem Speichervolumen vorgesehen. Im Betrieb trennt der Abscheider Leichtflüssigkeiten und/oder Fette von der zugeführten Flüssigkeit und das Abscheidevolumen sammelt die getrennten Leichtflüssigkeiten und/oder Fette. Gemäß der Erfindung ist zumindest ein Schichtstärkensensor zum Messen einer Schichtstärke zumindest einer Trennschicht im Abscheidevolumen angeordnet. Eine Steuereinheit ist dazu ausgebildet, die Hebeanlage zum Fördern von Flüssigkeiten aus dem Speichervolumen in Abhängigkeit der erfassten Schichtstärke (auch: Schichtdicke) freizugeben oder zu verriegeln. Es ist möglich, Abscheidevolumen und Speichervolumen in zwei separaten Behältern auszubilden, die fluidisch miteinander verbunden sind. Die Steuereinheit ist dazu konfiguriert, die Hebeanlage elektronisch freizugeben, wenn die Schichtstärke der im Abscheidevolumen befindlichen Trennschicht kleiner ist als ein vorgebbarer oder vorgegebener, kritischer Schwellwert.

Entgegen den bisher bekannten Ausführungen von selbsttätigen Verschlusseinrichtungen für Reinigungsanlagen der eingangs genannten Art mittels Schwimmer oder Zulaufsperren wird eine neue Reinigungsanlage ohne Schwimmer angegeben und als Verschlussorgan eine Hebeanlage vorgesehen. Schwimmer und Zulaufsperren sind in ihrem Grundzustand stets "offen", das bedeutet, dass zuströmende Flüssigkeit, insbesondere zuströmendes Abwasser, nach der Behandlung im gleichen Maße wieder durch derartig ausgebildete Verschlusseinrichtungen abfließen kann. Im Gegensatz dazu sind Hebeanlagen, bei denen eine Pumpe aktiv betrieben werden muss, um einen Ablauf zu erzeugen, in ihrem Grundzustand stets geschlossen. Dies wird gemäß der Erfindung ausgenutzt, indem die Hebeanlage mit einer Steuereinheit gekoppelt wird, die eine Aktivierung der Hebeanlage in Abhängigkeit der sensorisch erfassten Schichtdicke und gegebenenfalls von etwaig erfassten oder erkannten Fehlern freigibt bzw. sperrt. Liegen derartig erfasste oder erkannte Betriebsparameter in einem Normalbereich, so erfolgt eine elektronische Freigabe und das gereinigte Wasser kann mit Hilfe der Hebeanlage aus dem Speichervolumen in das angeschlossene Kanalsystem gepumpt werden. Andernfalls erfolgt eine elektronische Sperrung bzw. Blockierung der Hebeanlage.

Als Trennschicht im Sinne der vorliegenden Spezifizierung wird insbesondere eine Schicht angesehen, deren Bestandteile eine geringere Dichte als Wasser aufweisen. Insbesondere versteht man als Trennschicht die Linie, in der eine leichtere und eine schwere Flüssigkeit aneinander treffen. Also zum Beispiel die Linie die sich zwischen Öl und Wasser bildet - man kann es auch als Phasengrenze beschreiben. Eine derartige Trennschicht sammelt sich somit stets oberhalb des Wassers an. Die Trennschicht muss nicht zwangsläufig homogen sein und kann insbesondere verschiedene Bestandteile aufweisen, deren Dichten kleiner sind als die Dichte von Wasser. Die Trennschicht kann beispielsweise von verschiedenen Leichtflüssigkeiten, Mineralölbestandteilen, Ölen und/oder Fetten gebildet sein. Der Schichtstärkensensor erfasst die Schichtstärke der gesamten Schicht, die ein Maß für das Gesamtvolumen der abgeschiedenen Leichtflüssigkeiten bzw. Fette ist. Da die Dichte der die Trennschicht bildenden Bestandteile nicht maßgeblich für die Erfassung der Schichtstärke ist, kann eine zuverlässige Erfassung des abgeschiedenen Gesamtvolumens insbesondere in Anwendungsfällen erfolgen, in denen beispielsweise ein heterogenes Gemisch von Leichtflüssigkeiten abgeschieden werden soll.

Die Hebeanlage umfasst insbesondere zumindest eine Pumpe und eine Hebeleitung, um das gereinigte Abwasser aus dem Speichervolumen zu pumpen. Die Verwendung von Hebeanlage zu diesem Zweck ist insbesondere auch im Falle von Stromausfällen vorteilhaft, da dann die Hebeanlage nicht in Betrieb genommen werden kann, und somit Betriebsstörungen vermieden werden können.

In Ausgestaltungen sind bzw. ist im Abscheidevolumen und/oder im Speichervolumen ein Drucksensor zum Messen des Füllstandes (auch: Pegel, Flüssigkeitspegel) angeordnet. Die Steuereinheit ist in derartigen Ausführungen dazu ausgebildet, die Hebeanlage zum Fördern von Flüssigkeiten aus dem Speichervolumen in Abhängigkeit des erfassten Füllstandes freizugeben oder zu verriegeln. Der Drucksensor ist beispielsweise als Pegelsensor ausgeführt und erfasst vorzugsweise den hydrostatischen Druck der ihn umgebenden Flüssigkeit bzw. des ihn umgebenden Abwassers, der ein Maß für den Füllstand im Abscheidevolumen bzw. im Speichervolumen ist. Auf diese Weise kann sichergestellt werden, dass die Hebeanlage erst dann zum Fördern von gereinigtem Abwasser aus dem Speichervolumen freigegeben wird, wenn sich im Speichervolumen gereinigtes Abwasser in ausreichendem Volumen angesammelt hat. Alternativ oder zusätzlich wird mit Hilfe eines Drucksensors der Pegelstand im Abscheidevolumen überwacht. Dies ist insbesondere in Ausgestaltungen sinnvoll, in denen Abwasser bei zu hohem Pegelstand direkt vom Abscheidevolumen in das Speichervolumen fließen kann, also beispielsweise, wenn das Abscheidevolumen und das Speichervolumen von zwei Bereichen eines monolithischen Abscheidebeckens gebildet sind, die voneinander durch eine Trennwand getrennt sind. In solchen Fällen ist vorgesehen, dass die Steuereinheit die Hebeanlage automatisch verriegelt.

In Ausgestaltungen ist am Ablauf zum Abscheidevolumen eine elektronisch verriegelbare Sperre vorgesehen. Die Steuereinheit ist in derartigen Ausgestaltungen vorzugsweise dazu ausgebildet, die Sperre in Abhängigkeit der erfassten Schichtstärke und/oder in Abhängigkeit des erfassten Füllstandes, insbesondere im Abscheidevolumen, freizugeben oder zu verriegeln. Die Steuereinheit kann ein auslesbares elektronisches Betriebstagebuch umfassen. Mit Hilfe des Schichtstärkensensors kann insbesondere erfasst werden, ob das abgeschiedene Gesamtvolumen bereits das maximale Fassungsvermögen erreicht hat und der Abscheider entsprechend geleert werden muss. In diesem Fall bietet sich eine automatische Unterbrechung des Abflusses an. Im Falle eines zu hohen Flüssigkeitspegels im Abscheidevolumen kann beispielsweise ein weiterer Ablauf von Wasser automatisch unterbunden werden.

In Ausgestaltungen ist das Abscheidevolumen und das Speichervolumen durch zwei separate Abscheidebehälter, insbesondere aus Beton, Polymerbeton, Stahlbeton, Kunststoff, Metall, GFK realisiert.

In hierzu alternativen Ausgestaltungen ist das Abscheidevolumen und das Speichervolumen von zwei voneinander abgetrennten Bereichen eines Abscheidebehälters, die durch eine Trennwand sind, gebildet. Auch in diesem Fall besteht der Abscheidebehälter und/oder die Trennwand vorzugsweise aus Beton Polymerbeton, Stahlbeton, Kunststoff, Metall, GFK. Der Abscheidebehälter und die Trennwand können vorzugsweise monolithisch ausgebildet sein.

In Ausgestaltungen ist der Schichtstärkensensor zur Messung der Leitfähigkeit oder Polarität, insbesondere des Dipolmoments, der ihn umgebenden Flüssigkeit ausgebildet. Mit Hilfe derartiger Messungen kann die den Schichtstärkensensor umgebende Flüssigkeit hinreichend charakterisiert werden. Insbesondere ist es möglich, polare Flüssigkeiten, wie etwa Wasser, von unpolaren Flüssigkeiten, wie etwa Fetten, Ölen oder andere Leichtflüssigkeiten, voneinander zu unterscheiden. Vorzugsweise umfasst der Schichtstärkensensor zur Erfassung der Schichtstärke der Trennschicht mehrere räumlich verteilt angeordnete Einzelsensoren, die zur Messung der Leitfähigkeit oder Polarität, insbesondere des Dipolmoments, der jeweils umgebenden Flüssigkeit ausgebildet sind.

In Ausgestaltungen weist der Abscheider zumindest ein Koaleszenzelement, das insbesondere zumindest ein Drahtgestrick oder ein Lochblech umfasst, auf. Das Koaleszenzelement dient insbesondere dazu, die Anlagerung von Leichtflüssigkeitsbestandteilen, Fetten und/oder Ölen zu unterstützen und weist hierzu eine geeignete Oberflächenbeschaffenheit auf. In Ausgestaltungen ist das Koaleszenzelement zumindest abschnittsweise von einem oelophilen Material gebildet und/oder weist zumindest abschnittsweise eine oelophile Beschichtung auf.

In Ausgestaltungen ist eine Warneinrichtung zur Ausgabe eines akustischen und/oder visuellen Warnsignals vorgesehen. Die Warneinrichtung ist dazu ausgebildet, das Warnsignals in Abhängigkeit der erfassten Schichtstärke und/oder des erfassten Füllstandes auszugeben.

Bei einem Verfahren zum Betrieb einer Reinigungsanlage, insbesondere für Abwasser, zur Abscheidung von Leichtflüssigkeit und/oder Fetten, zum Betrieb der vorstehend beschriebenen Reinigungsanlage, ist zumindest ein Abscheidevolumen und ein Speichervolumen vorgesehen, welche von einem oder von mehreren Abscheidebehälter gebildet sind. Das Abscheidevolumen und das Speichervolumen sind über einen Abscheider zum Abscheiden von Leichtflüssigkeiten und/oder Fetten fluidisch miteinander verbunden. Zu reinigende Flüssigkeiten, insbesondere Abwasser, werden dem Abscheidevolumen zugeführt. Verunreinigungen, insbesondere in Form von Leichtflüssigkeiten und/oder Fetten, werden über den Abscheider abgeschieden und gereinigte Flüssigkeit wird im Speichervolumen gesammelt. Gemäß der Erfindung wird eine Hebeanlage zum Fördern der gereinigten Flüssigkeit aus dem Speicherbereich elektronisch freigegeben, wenn die Schichtstärke einer im Abscheidevolumen befindlichen Trennschicht kleiner ist als ein vorgebbarer oder vorgegebener, kritischer Schwellwert.

Im Regelbetrieb (auch: Normbetrieb, Normalbetrieb), durchläuft das zu reinigende Wasser den Bereich des Abscheiders und wird im Speichervolumen der Hebeanlage gesammelt, um von dort in das angeschlossene Kanalsystem gepumpt zu werden. Als Kriterium dafür, dass die Reinigungsanlage im Regelbetrieb betrieben werden kann, gilt insbesondere, dass die das bereits abgeschiedene Gesamtvolumen charakterisierende Schichtstärke der Trennschicht unterhalb des kritischen Schwellwert liegt. Der kritische Schwellwert entspricht insbesondere der maximalen Speichermenge für abgeschiedenen Leichtflüssigkeiten bzw. Fetten. Im Regelbetrieb sollte die erfasste Schichtstärke stets unterhalb des kritischen Schwellwerts liegen, da standardmäßig der Abscheider regelmäßig entleert werden muss. Erreicht die erfasste Schichtstärke dennoch den vorgegeben oder vorgebbaren, kritischen Schwellwert, so erfolgt eine elektronische Sperrung der Hebeanlage. Der Betrieb wird erst nach benutzerseitiger Freigabe entsperrt. Es ist vorgesehen, derartige Ereignisse in einem Protokoll der Steuereinheit zu speichern. Die manuelle Freigabe erfolgt erst dann, wenn nach einer Sichtkontrolle der Regelbetrieb festgestellt worden ist.

Das elektronische Sperren der Hebeanlage hat unter anderem auch den Vorteil, dass der normale Betriebszustand (Regelbetrieb) benutzerseitig durch einfaches Zurücksetzen der Steuereinheit wiederhergestellt werden kann. Dies ist bei den bekannten Verriegelungseinrichtungen nicht der Fall, da im Allgemeinen hierzu ein manueller Eingriff mit anschließender Entleerung erforderlich ist.

In Ausgestaltungen wird die Hebeanlage zum Fördern der gereinigten Flüssigkeit aus dem Speicherbereich freigegeben, wenn ein erfasster Füllstand im Abscheidevolumen und/oder im Speichervolumen innerhalb eines Normbereichs liegt. Beispielsweise wird gereinigtes Abwasser im Speichervolumen der Hebeanlage gesammelt, bis der dort installierte Drucksensor (z.B. Pegelsonde) ein ausreichendes Volumen meldet, so dass das gereinigte Wasser in die angeschlossene Rohrleitung gepumpt werden kann. In anderen Fällen kann die Situation entstehen, dass der Flüssigkeitspegel im Abscheidevolumen durch einen zu großen Zufluss zu stark ansteigt. Dies ist insbesondere bei Ausführungen kritisch, die in einem einzigen Abscheidebecken realisiert sind, also wenn Abscheider und Hebeanlage beispielsweise in einem monolithischen Abscheidebehälter umgesetzt sind, der durch eine Trennwand in zwei Funktionsbereiche geteilt ist. Hier besteht die Gefahr, dass durch einen zu großen Zufluss der Flüssigkeitspegel im Abscheidevolumen so weit ansteigt, dass bereits abgeschiedene Leichtflüssigkeit über die Trennwand steigt. In diesem Fall sendet der Sensor im Abscheidevolumen ein entsprechendes Signal an die Steuereinheit, die daraufhin die Hebeanlage elektronisch verriegelt und erst nach benutzerseitiger Bestätigung wieder für den Regelbetrieb freigibt. Vorzugsweise werden derartige Fehlermeldungen im Protokoll der Steuereinheit protokolliert.

In Ausgestaltungen wird ein Warnsignal ausgegeben, wenn die erfasste Schichtstärke einen vorgegebenen oder vorgebaren Grenzwert, der unterhalb des kritischen Schwellwerts liegt, erreicht oder überstiegt. Die Ausgabe von derartigen Warnsignalen soll insbesondere verhindern, dass bereits abgeschiedene Leichtflüssigkeit unkontrolliert in das Speichervolumen gelangen kann, wenn die maximale Abscheidemenge im Abscheider bereits erreicht wurde.

In Ausgestaltungen korrespondiert der kritische Schwellwert zu einer kritischen Schichtstärke mit einem entsprechenden kritischen Volumen. Das kritische Volumen entspricht insbesondere der maximalen Abscheidemenge, die das Abscheidevolumen fassen kann. Der zur Ausgabe des akustischen und/oder optischen Warnsignals maßgebliche Grenzwert korrespondiert entsprechend vorzugsweise zu einem Volumen, das 80% des kritischen Volumens entspricht. Auf diese Weise wird sichergestellt, dass ein Alarm ausgegeben wird, wenn das abgeschiedenen Gesamtvolumen in etwa 80% der maximalen Abscheidemenge erreicht hat. Somit bleibt dem Betreiber im Regelfall noch ausreichend Zeit, das Abscheidevolumen zu entleeren, bevor es zu einem Abschluss aufgrund des Erreichens der maximalen Abscheidemenge kommt.

Die Erfindung wird nachstehend auch hinsichtlich weiterer Merkmale und Vorteile anhand der Beschreibung von Ausführungsbeispielen und unter Bezugnahme auf die beiliegenden, schematischen Zeichnungen näher erläutert. Es zeigen
FIG. 1 eine Reinigungsanlage im geöffneten Zustand in einer Draufsicht;
FIG. 2 die Reinigungsanlage der FIG. 1 in einer Querschnittdarstellung;
FIG. 3 die Reinigungsanlage der FIG. 1 in einer weiteren Schnittdarstellung;
FIG. 4 die Reinigungsanlage der FIG. 1 in einer weiteren Schnittdarstellung.

Einander entsprechende Teile und Komponenten sind in allen Figuren mit den gleichen Bezugszeichen versehen.

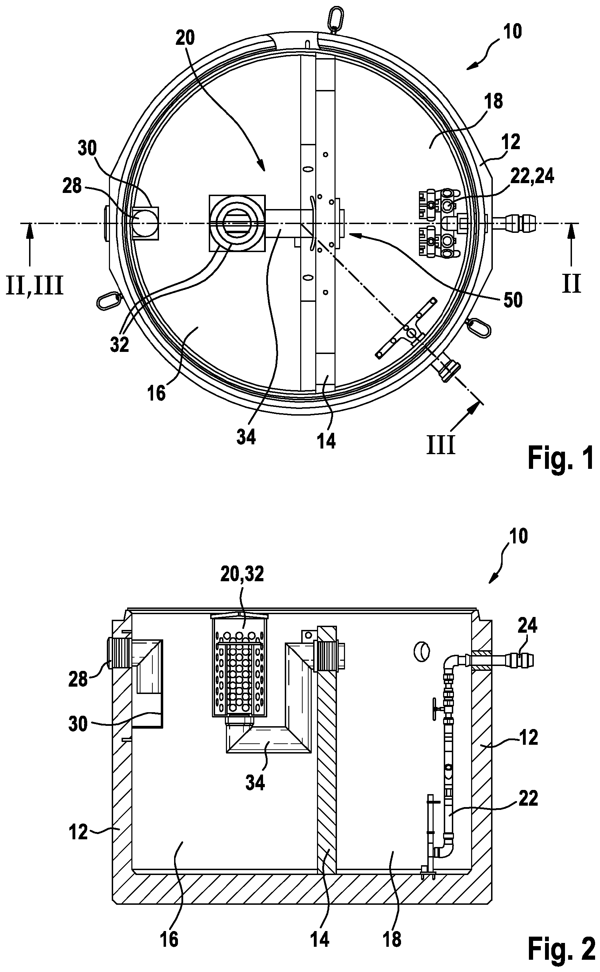

FIG. 1 zeigt eine Reinigungsanlage 10 zur Abscheidung von Leichtflüssigkeit und/oder Fetten in Abwasser in einer Draufsicht. Die Reinigungsanlage umfasst einen monolithischen Abscheidebehälter 12 aus Beton, der im eingebauten Zustand von einem nicht näher dargestellten Deckel, der ebenfalls aus Beton besteht, verschlossen ist. Eine Trennwand 14 unterteilt das Innere des Abscheidebehälters 12 in ein Abscheidevolumen 16 und ein Speichervolumen 18.

Das Abscheidevolumen 16 und das Speichervolumen 18 sind über einen Abscheider 20 zum Abscheiden von Leichtflüssigkeiten und/oder Fetten fluidisch verbunden. Im Speichervolumen 18 ist eine Hebeleitung 22 einer Hebeanlage 24 zum Fördern von gereinigtem Abwasser aus dem Speichervolumen 18 angeordnet. Zum Fördern des gereinigten Abwassers ist eine nicht näher dargestellte Pumpe an der Hebeleitung 22 angeschlossen.

Abwasser gelangt über einen Zulauf 26 in das Abscheidevolumen 16. Der Zulauf 28 ist mit einem Prallblech 30 versehen, damit das einströmende Abwasser sich beruhigen kann und sich insbesondere Festkörperbestandteile durch Sedimentation absetzen können.

Im Abscheidevolumen 16 ist zumindest ein Schichtstärkensensor 52 zum Messen einer Schichtstärke zumindest einer aus bereits abgeschiedenen Leichtflüssigkeits- oder Fettbestandteilen bestehenden Trennschicht angeordnet. Der Schichtstärkensensor 52 ist zur Messung der Leitfähigkeit oder Polarität, insbesondere des Dipolmoments, der ihn umgebenden Flüssigkeit ausgebildet. Hierbei weist der Schichtstärkensensor 52 eine gewisse räumliche Auflösung auf, so dass die Dicke bzw. Schichtstärke der Trennschicht erfassbar ist.

Eine nicht näher dargestellte Steuereinheit ist dazu ausgebildet, die Hebeanlage 24 zum Fördern von Flüssigkeit aus dem Speichervolumen 16 in Abhängigkeit der erfassten Schichtstärke freizugeben oder zu verriegeln. Die Hebeanlage 24 ist somit mit der Steuereinheit derart gekoppelt, dass eine elektronische Freigabe bzw. Sperrung zur Aktivierung der Hebeanlage 24 in Abhängigkeit der sensorisch erfassten Schichtdicke bzw. Schichtstärke und gegebenenfalls in Abhängigkeit von erkannten Fehlern erfolgt.

Der Abscheider 20 ist in dem gezeigten Ausführungsbeispiel als Koaleszenzabscheider ausgeführt und umfasst ein im Wesentlichen zylindrisches Koaleszenzelement 32, die von Lochblechen gebildet und zueinander konzentrisch angeordnet sind. Der Abscheider 20 umfasst weiter ein Überlaufrohr 34, welches die fluidische Verbindung zwischen dem Abscheidevolumen 16 und dem Speichervolumen 18 bereitstellt.

Im Abscheidevolumen 16 und im Speichervolumen 18 sind Drucksensoren zum Messen des Füllstandes in den jeweiligen Bereichen angeordnet. Die Steuereinheit steht mit diesen Drucksensoren drahtgebunden oder drahtlos in einer Wirkverbindung und ist dazu ausgebildet, die Hebeanlage 24 zum Fördern von Flüssigkeiten aus dem Speichervolumen 18 in Abhängigkeit der jeweilig erfassten Füllstände freizugeben oder zu verriegeln. Die Drucksensoren sind zur Erfassung des hydrostatischen Umgebungsdrucks in den jeweiligen Bereichen ausgebildet.

Am Ablauf 22 zum Abscheidevolumen 16 ist ferner eine nicht näher dargestellte, elektronisch verriegelbare Sperre vorgesehen. Die Steuereinheit ist dazu ausgebildet, die Sperre in Abhängigkeit der erfassten Schichtstärke und in Abhängigkeit der erfassten Füllstände im Abscheidevolumen 16 und im Speichervolumen 18 freizugeben oder zu verriegeln.

Ferner ist eine nicht näher dargestellte Warneinrichtung zur Ausgabe eines akustischen und/oder visuellen Warnsignals vorgesehen. Die Warneinrichtung ist dazu ausgebildet, das Warnsignals in Abhängigkeit der erfassten Schichtstärke und/oder des erfassten Füllstandes auszugeben.

Ein Probenahmebereich 50 ist zwischen Abscheidevolumen 16 und Speichervolumen 18 vorgesehen. Der Probenahmebereich ist in die Trennwand 14 integriert. In dem Fall, dass Abscheidevolumen 16 und Speichervolumen 18 in separaten Behältern angeordnet sind, ist der Probenahmebereich ebenfalls als separater Behälter ausgeführt, der fluidisch mit Abscheidevolumen 16 und Speichervolumen verbunden ist.

FIG. 2 zeigt eine Querschnittsdarstellung der Reinigungsanlage 10. Der Verlauf des gezeigten Querschnitts ist in FIG. 1 gezeigt und mit II bezeichnet.

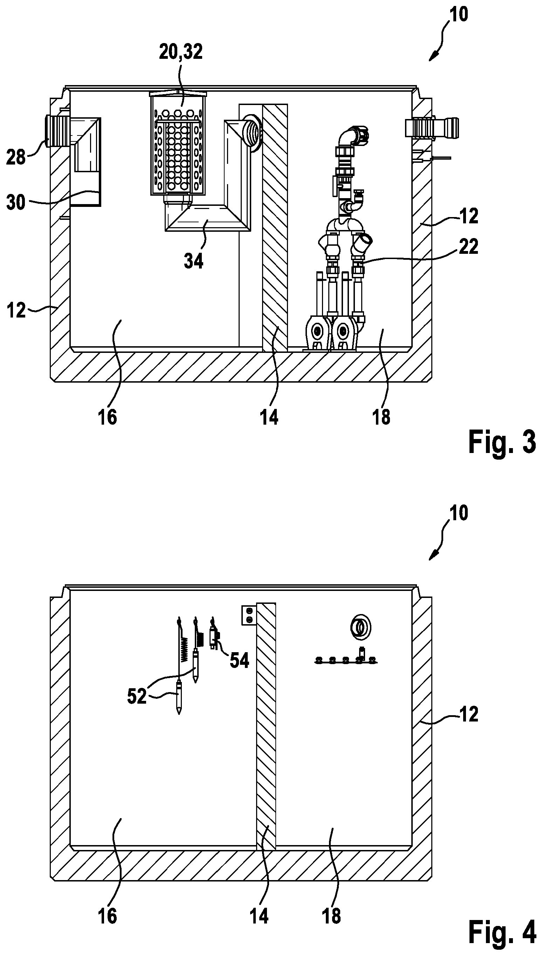

FIG. 3 zeigt eine weitere Schnittdarstellung der Reinigungsanlage 10. Der Verlauf des gezeigten Schnitts ist in FIG. 1 gezeigt und mit III bezeichnet.

FIG. 4 zeigt eine weitere Schnittdarstellung der Reinigungsanlage 10, in der insbesondere die Lage der Schichtstärkensensoren 52 und die Lage einer Aufstausonde 54 im Abscheidevolumen 16 gezeigt sind.

Beim Betrieb der Reinigungsanlage 10 werden Leichtflüssigkeiten und/oder Fette vom zugeführten Abwasser mit Hilfe des Abscheiders 20 getrennt und im Abscheidevolumen 16 gesammelt. Hiervon gereinigtes Abwasser gelangt über das Überlaufrohr 34 in das Speichervolumen 18 und sammelt sich dort. Die Hebeanlage 24 wird in Abhängigkeit der mittels der Drucksensoren und des Schichtstärkensensors 52 erfassten sensorischen Daten von der Steuereinheit zum Fördern des gereinigten Abwassers aus dem Speicherbereich 18 elektronisch freigegeben oder gesperrt. Eine Freigabe erfolgt insbesondere dann, wenn die Schichtstärke einer im Abscheidevolumen 18 befindlichen Trennschicht kleiner ist als ein vorgebbarer oder vorgegebener, kritischer Schwellwert und die übrigen erfassten Daten auf keine andere Betriebsstörung hinweisen. Im Bereich des Überlaufrohres 34 kann die Probenahme erfolgen, bzw. Probenahmebereich 50 angeordnet werden.

Im Regelbetrieb durchläuft das zu reinigende Wasser den Bereich des Abscheiders 20 und wird im Speichervolumen 18 der Hebeanlage 24 gesammelt und von dort in das angeschlossene Kanalsystem gepumpt. Ein notwendiges, jedoch nicht zwangsläufig hinreichendes Kriterium für den Regelbetrieb der Reinigungsanlage 10 im Regelbetrieb ist, dass das bereits abgeschiedene Gesamtvolumen an Leichtflüssigkeiten, Ölen und/oder Fetten unterhalb der maximalen Abscheidemenge liegt, die das Abscheidevolumen 16 aufnehmen kann. Die maximale Abscheidemenge entspricht dem kritischen Schwellwert für die Schichtstärke der Trennschicht.

Im Regelbetrieb sollte die Schichtstärke der Trennschicht im Abscheidevolumen 16 stets unterhalb des kritischen Schwellwerts liegen, da zuvor ein Alarm ausgegeben wird, der den Betreiber dazu anweist, das Abscheidevolumen zu entleeren. Erreicht die erfasste Schichtstärke dennoch den kritischen Schwellwert, so erfolgt eine elektronische Sperrung der Hebeanlage 24. Der Betrieb wird erst nach benutzerseitiger Freigabe entsperrt. Es ist vorgesehen, dies in einem Protokoll der Steuereinheit zu speichern.

Für den Regelbetrieb ist im Allgemeinen zusätzlich notwendig, dass der Füllstand im Abscheidevolumen 16 und im Speichervolumen 18 innerhalb eines Normbereichs liegen. Insbesondere muss der Pegel im Speichervolumen 18 hoch genug sein, damit die Hebeanlage 24 aktiviert werden kann. Der Flüssigkeitspegel im Abscheidevolumen 16 kann beispielsweise durch einen zu großen Zufluss zu hoch sein, so dass die Gefahr besteht, dass bereits abgeschiedene Leichtflüssigkeit oder bereits abgeschiedenes Fett über die Trennwand 14 in den Speicherbereich 18 gelangt. In solchen Fällen ist vorgesehen, dass die Steuereinheit die Hebeanlage 24 automatisch elektronisch verriegelt und erst nach benutzerseitiger Bestätigung wieder für den Regelbetrieb freigibt. Derartige Betriebsstörungen werden im Protokoll der Steuereinheit protokolliert.

Das bereits erwähnte Warnsignal wird ausgegeben, wenn die erfasste Schichtstärke der Trennschicht einen vorgegebenen oder vorgebaren Grenzwert, der unterhalb des kritischen Schwellwerts liegt, erreicht oder überstiegt. Wie bereits erwähnt, entspricht der kritische Schwellwert einer maximalen Abscheidemenge für Leichtflüssigkeiten und/oder Fette. Der zur Ausgabe des akustischen und/oder optischen Warnsignals maßgebliche Grenzwert korrespondiert vorzugsweise zu einem Volumen, das 80% der maximalen Abscheidemenge entspricht. Somit bleibt dem Betreiber bei Ausgabe des Warnsignals im Regelfall noch ausreichend Zeit, das Abscheidevolumen 16 zu entleeren, bevor es zu einem Abschluss aufgrund des Erreichens der maximalen Abscheidemenge kommt.

## Patentansprüche

1. Reinigungsanlage (10), insbesondere für Abwasser, zur Abscheidung von Leichtflüssigkeit und/oder Fetten, mit einem oder mehreren, zumindest ein Abscheidevolumen (16) und ein Speichervolumen (18) bildenden Abscheidebehälter (12), wobei das Abscheidevolumen (16) zumindest einen Zulauf (28) für zu reinigende Flüssigkeiten, insbesondere Abwasser aufweist und das Abscheidevolumen (16) und das Speichervolumen (18) über einen Abscheider (20) zum Abscheiden von Leichtflüssigkeiten und/oder Fetten fluidisch verbunden sind, wobei eine Hebeanlage (24) zum Fördern von Flüssigkeit aus dem Speichervolumen (18) vorgesehen ist,
**dadurch gekennzeichnet, dass** im Betrieb der Abscheider (20) Leichtflüssigkeiten und/oder Fette von der zugeführten Flüssigkeit trennt und das Abscheidevolumen (16) die getrennten Leichtflüssigkeiten und/oder Fette sammelt, wobei zumindest ein Schichtstärkensensor zum Messen einer Schichtstärke zumindest einer Trennschicht im Abscheidevolumen (16) angeordnet ist und eine Steuereinheit dazu ausgebildet ist, die Hebeanlage (24) zum Fördern von Flüssigkeiten aus dem Speichervolumen (18) in Abhängigkeit der erfassten Schichtstärke freizugeben oder zu verriegeln, wobei die Steuereinheit dazu konfiguriert ist, die Hebeanlage (24) elektronisch freizugeben, wenn die Schichtstärke der im Abscheidevolumen (16) befindlichen Trennschicht kleiner ist als ein vorgebbarer oder vorgegebener, kritischer Schwellwert.

2. Reinigungsanlage (10) nach Anspruch 1, **dadurch gekennzeichnet, dass** im Abscheidevolumen (16) und/oder im Speichervolumen (18) ein Sensor, insbesondere Drucksensor, Füllstandsensor, Vibrationssensor, kapazitiver Sensor oder thermischer Sensor, zum Messen des Füllstandes angeordnet ist und die Steuereinheit dazu ausgebildet ist, die Hebeanlage (24) zum Fördern von Flüssigkeiten aus dem Speichervolumen (18) in Abhängigkeit des erfassten Füllstandes freizugeben oder zu verriegeln.

3. Reinigungsanlage (10) nach Anspruch 1 oder 2, **dadurch gekennzeichnet, dass** an einem Ablauf und/oder Zulauf (22, 24, 28) eine elektronisch verriegelbare Sperre vorgesehen ist und die Steuereinheit dazu ausgebildet ist, die Sperre in Abhängigkeit der erfassten Schichtstärke und/oder in Abhängigkeit des erfassten Füllstandes, insbesondere im Abscheidevolumen (16) und/oder im Speichervolumen (18), freizugeben oder zu verriegeln.

4. Reinigungsanlagen (10) nach einem der vorhergehenden Ansprüche, **dadurch gekennzeichnet, dass** das Abscheidevolumen (16) und das Speichervolumen (20) durch zwei separate Abscheidebehälter, insbesondere aus Beton, Polymerbeton, Metall, GFK oder Kunststoff, realisiert sind.

5. Reinigungsanlage (10) nach einem der Ansprüche 1 bis 3, **dadurch gekennzeichnet, dass** das Abscheidevolumen (16) und das Speichervolumen (18) von zwei durch eine Trennwand (14) voneinander abgetrennten Bereichen eines Abscheidebehälters (12), insbesondere aus Beton, Polymerbeton, Metall, GFK oder Kunststoff, gebildet sind.

6. Reinigungsanlage (10) nach einem der vorhergehenden Ansprüche, **dadurch gekennzeichnet, dass** der Schichtstärkensensor zur Messung der Leitfähigkeit oder Polarität der ihn umgebenden Flüssigkeit ausgebildet ist.

7. Reinigungsanlage (10) nach einem der vorhergehenden Ansprüche, **dadurch gekennzeichnet, dass** der Abscheider (20) zumindest ein Koaleszenzelement (32), das insbesondere zumindest ein Drahtgestrick oder ein Lochblech umfasst, aufweist.

8. Reinigungsanlage (10) nach einem der vorhergehenden Ansprüche, **dadurch gekennzeichnet, dass** eine Warneinrichtung zur Ausgabe eines akustischen und/oder visuellen Warnsignals vorgesehen ist, wobei die Warneinrichtung dazu ausgebildet ist, das Warnsignals in Abhängigkeit der erfassten Schichtstärke und/oder des erfassten Füllstandes auszugeben.

9. Verfahren zum Betrieb einer Reinigungsanlage (10), insbesondere für Abwasser, zur Abscheidung von Leichtflüssigkeit und/oder Fetten nach einem der vorhergehenden Ansprüche, mit einem oder mehreren, zumindest ein Abscheidevolumen (16) und ein Speichervolumen (18) bildenden Abscheidebehälter (12), die über einen Abscheider (20) zum Abscheiden von Leichtflüssigkeiten und/oder Fetten fluidisch verbunden sind, wobei zu reinigende Flüssigkeiten, insbesondere Abwasser, dem Abscheidevolumen (16) zugeführt werden, Verunreinigungen in Form von Leichtflüssigkeiten und/oder Fetten über den Abscheider (20) abgeschieden werden, die abgeschiedenen Leichtflüssigkeiten und/oder Fette in dem Abscheidevolumen (16) gesammelt werden und gereinigte Flüssigkeit im Speichervolumen (18) gesammelt wird, wobei eine Hebeanlage (24) zum Fördern der gereinigten Flüssigkeit aus dem Speicherbereich (18) elektronisch freigegeben wird, wenn die Schichtstärke einer im Abscheidevolumen (16) befindlichen Trennschicht kleiner ist als ein vorgebbarer oder vorgegebener, kritischer Schwellwert.

10. Verfahren nach Anspruch 9, **dadurch gekennzeichnet, dass** die Hebeanlage (24) zum Fördern der gereinigten Flüssigkeit aus dem Speicherbereich (18) freigegeben wird, wenn ein erfasster Füllstand im Abscheidevolumen (16) und/oder im Speichervolumen (18) innerhalb eines Normbereichs liegt.

11. Verfahren nach Anspruch 9, **dadurch gekennzeichnet, dass** ein Warnsignal ausgegeben wird, wenn die erfasste Schichtstärke einen vorgegebenen oder vorgebaren Grenzwert, der unterhalb des kritischen Schwellwerts liegt, erreicht oder übersteigt.

12. Verfahren nach Anspruch 11, **dadurch gekennzeichnet, dass** der kritische Schwellwert zu einer kritischen Schichtstärke mit einem entsprechenden kritischen Volumen korrespondiert, und der Grenzwert zu einem Volumen korrespondiert, das 80% des kritischen Volumens entspricht.

## Claims

1. A cleaning system (10), in particular for waste water, for separating light liquid and/or fats, with one or more separating containers (12) forming at least one separating volume (16) and one storage volume (18), wherein the separating volume (16) has at least one inlet (28) for to-be-cleaned liquids, in particular waste water, and the separating volume (16) and the storage volume (18) are fluidically connected via a separator (20) for separating light liquids and/or fats, wherein a lifting system (24) is provided for transporting liquid from the storage volume (18),
**characterised in that**, during operation, the separator (20) separates light liquids and/or fats from the supplied liquid and the separating volume (16) collects the separated light liquids and/or fats, wherein at least one layer thickness sensor for measuring a layer thickness of at least one separating layer is arranged in the separating volume (16) and a control unit is designed to release or lock the lifting system (24) for transporting liquids from the storage volume (18) as a function of the sensed layer thickness, wherein the control unit is configured to release the lifting system (24) electronically if the layer thickness of the separating layer located in the separating volume (16) is smaller than a predeterminable or predetermined critical threshold.

2. The cleaning system (10) according to claim 1, **characterised in that** a sensor, in particular a pressure sensor, filling level sensor, vibration sensor, capacitive sensor or thermal sensor, for measuring the filling level is arranged in the separating volume (16) and/or in the storage volume (18), and the control unit is designed to release or lock the lifting system (24) for transporting liquids from the storage volume (18) as a function of the sensed filling level.

3. The cleaning system (10) according to claim 1 or 2, **characterised in that** an electronically lockable barrier is provided at an outlet and/or inlet (22, 24, 28), and the control unit is designed to release or lock the barrier as a function of the sensed layer thickness and/or as a function of the sensed filling level, in particular in the separating volume (16) and/or in the storage volume (18).

4. The cleaning system (10) according to one of the preceding claims, **characterised in that** the separating volume (16) and the storage volume (20) are realised by two separate separating containers, in particular made of concrete, polymer concrete, metal, GRP or plastic.

5. The cleaning system (10) according to any of claims 1 to 3, **characterised in that** the separating volume (16) and the storage volume (18) are formed by two areas of a separating container (12) separated from one another by a partition wall (14), which are in particular made of concrete, polymer concrete, metal, GRP or plastic.

6. The cleaning system (10) according to one of the preceding claims, **characterised in that** the layer thickness sensor is designed for measuring the conductivity or the polarity of the liquid surrounding it.

7. The cleaning system (10) according to one of the preceding claims, **characterised in that** the separator (20) has at least one coalescence element (32), which in particular comprises at least one wire mesh or a perforated plate.

8. The cleaning system (10) according to one of the preceding claims, **characterised in that** a warning device is provided for outputting an acoustic and/or visual warning signal, wherein the warning device is designed to output the warning signal as a function of the sensed layer thickness and/or the sensed filling level.

9. A method for operating a cleaning system (10), in particular for waste water, for separating light liquid and/or fats according to one of the preceding claims, with one or more separating containers (12) forming at least one separating volume (16) and one storage volume (18), which are fluidically connected via a separator (20) for separating light liquids and/or fats, wherein to-be-cleaned liquids, in particular waste water, are fed into the separating volume (16), impurities in the form of light liquids and/or fats are separated via the separator (20), the separated light liquids and/or fats are collected in the separating volume (16) and cleaned liquid is collected in the storage volume (18), wherein a lifting system (24) for transporting the cleaned liquid out of the storage area (18) is released electronically if the layer thickness of a separating layer located in the separating volume (16) is smaller than a predeterminable or predetermined critical threshold.

10. The method according to claim 9, **characterised in that** the lifting system (24) is released for transporting the cleaned liquid from the storage area (18) if a sensed filling level in the separating volume (16) and/or in the storage volume (18) is within a nominal range.

11. The method according to claim 9, **characterised in that** a warning signal is output if the sensed layer thickness reaches or exceeds a predetermined or predeterminable limit which is below the critical threshold.

12. The method according to claim 11, **characterised in that** the critical threshold corresponds to a critical layer thickness with a respective critical volume, and the limit corresponds to a volume corresponding to 80 % of the critical volume.

## Revendications

1. Installation d'épuration (10), en particulier pour des eaux usées, pour la séparation de liquide léger et/ou de graisses, avec un ou plusieurs cuves de séparation (12) formant au moins un volume de séparation (16) et un volume d'accumulation (18), dans laquelle le volume de séparation (16) comporte au moins une arrivée (28) pour des liquides à épurer, en particulier des eaux usées, et le volume de séparation (16) et le volume d'accumulation (18) communiquent pour la circulation de fluide via un séparateur (20) pour la séparation de liquides légers et/ou de graisses, une installation de relevage (24) étant prévue pour transporter du liquide hors du volume d'accumulation (18), **caractérisée en ce que**, en fonctionnement, le séparateur (20) sépare des liquides légers et/ou des graisses du liquide amené et le volume de séparation (16) collecte les liquides légers et/ou les graisses séparés, au moins un capteur d'épaisseur de couche étant disposé pour mesurer une épaisseur de couche d'au moins une couche de séparation dans le volume de séparation (16) et une unité de commande étant conçue pour débloquer ou verrouiller l'installation de relevage (24) pour le transport de liquides hors du volume d'accumulation (18) en fonction de l'épaisseur de couche acquise, l'unité de commande étant configurée pour débloquer électroniquement l'installation de relevage (24) quand l'épaisseur de couche de la couche de séparation qui se trouve dans le volume de séparation (16) est inférieure à une valeur de seuil critique qui peut être ou est prédéfinie.

2. Installation d'épuration (10) selon la revendication 1, **caractérisée en ce qu'**un capteur, en particulier un capteur de pression, un capteur de niveau de remplissage, un capteur de vibrations, un capteur capacitif ou un capteur thermique, est disposé dans le volume de séparation (16) et/ou dans le volume d'accumulation (18) pour mesurer le niveau de remplissage et l'unité de commande est conçue pour débloquer ou verrouiller l'installation de relevage (24) pour le transport de liquides hors du volume d'accumulation (18) en fonction du niveau de remplissage acquis.

3. Installation d'épuration (10) selon la revendication 1 ou 2, **caractérisée en ce qu'**un barrage pouvant être verrouillé électroniquement est prévu sur une sortie et/ou une arrivée (22, 24, 28) et l'unité de commande est conçue pour débloquer ou verrouiller le barrage en fonction de l'épaisseur de couche acquise et/ou en fonction du niveau de remplissage acquis, en particulier dans le volume de séparation (16) et/ou dans le volume d'accumulation (18).

4. Installation d'épuration (10) selon l'une des revendications précédentes, **caractérisée en ce que** le volume de séparation (16) et le volume d'accumulation (20) sont formés par deux cuves de séparation séparées, en particulier en béton, en béton polymère, en métal, en plastique armé de fibres de verre ou en plastique.

5. Installation d'épuration (10) selon l'une des revendications 1 à 3, **caractérisée en ce que** le volume de séparation (16) et le volume d'accumulation (18) sont formés par deux zones d'une cuve de séparation (12), en particulier en béton, en béton polymère, en métal, en plastique armé de fibres de verre ou en plastique, séparées l'une de l'autre par une cloison (14).

6. Installation d'épuration (10) selon l'une des revendications précédentes, **caractérisée en ce que** le capteur d'épaisseur de couche est conçu pour mesurer la conductivité ou la polarité du liquide qui l'entoure.

7. Installation d'épuration (10) selon l'une des revendications précédentes, **caractérisée en ce que** le séparateur (20) comporte au moins un élément à coalescence (32) qui comprend en particulier au moins un grillage métallique tricoté ou une tôle perforée.

8. Installation d'épuration (10) selon l'une des revendications précédentes, **caractérisée en ce qu'**une installation d'avertissement destinée à émettre un signal d'avertissement acoustique et/ou visuel est prévue, l'installation d'avertissement étant conçue pour émettre le signal d'avertissement en fonction de l'épaisseur de couche acquise et/ou du niveau de remplissage acquis.

9. Procédé de pilotage d'une installation d'épuration (10), en particulier pour des eaux usées, pour la séparation de liquide léger et/ou de graisses selon l'une des revendications précédentes, avec une ou plusieurs cuves de séparation (12) formant au moins un volume de séparation (16) et un volume d'accumulation (18) qui communiquent pour la circulation de fluide via un séparateur (20) pour la séparation de liquides légers et/ou de graisses, dans lequel des liquides à épurer, en particulier des eaux usées, sont amenés au volume de séparation (16), des impuretés formées de liquides légers et/ou de graisses sont séparées par le séparateur (20), les liquides légers et/ou les graisses séparés sont accumulés dans le volume de séparation (16) et le liquide épuré est accumulé dans le volume d'accumulation (18), une installation de relevage (24) pour le transport du liquide épuré hors de la zone d'accumulation (18) étant débloquée électroniquement quand l'épaisseur de couche d'une couche de séparation qui se trouve dans le volume de séparation (16) est inférieure à un seuil critique qui peut être ou est prédéfini.

10. Procédé selon la revendication 9, **caractérisé en ce que** l'installation de relevage (24) pour le transport du liquide épuré hors de la zone d'accumulation (18) est débloquée quand un niveau de remplissage acquis dans le volume de séparation (16) et/ou dans le volume d'accumulation (18) se trouve dans une plage normée.

11. Procédé selon la revendication 9, **caractérisé en ce qu'**un signal d'avertissement est émis quand l'épaisseur de couche acquise atteint ou dépasse une limite qui est ou peut être prédéfinie et qui se situe en dessous du seuil critique.

12. Procédé selon la revendication 11, **caractérisé en ce que** le seuil critique correspond à une épaisseur de couche critique avec un volume critique correspondant et la limite correspond à un volume qui correspond à 80 % du volume critique.
